# EUROPEAN PATENT SPECIFICATION

(11) **EP 3 733 052 B1**
(45) Date of publication and mention of the grant of the patent: **14.05.2025**
(21) Application number: 19754674.0
(22) Date of filing: 24.01.2019
(51) Int. Cl.: A61B 5/00, G16H 40/40, G16H 40/67, A61B 5/1486, A61B 5/145

(54) **METHOD FOR MANAGING BIOMETRIC INFORMATION USING SENSOR USAGE INFORMATION**
VERFAHREN ZUR VERWALTUNG BIOMETRISCHER INFORMATIONEN UNTER VERWENDUNG VON SENSORNUTZUNGSINFORMATIONEN
PROCÉDÉ DE GESTION D'INFORMATIONS BIOMÉTRIQUES FAISANT APPEL À DES INFORMATIONS D'UTILISATION DE CAPTEURS

(30) Priority: 14.02.2018 KR 20180018399
(43) Date of publication of application: 04.11.2020
(73) Proprietor: i-Sens, Inc., Seoul 06646 (KR)
(72) Inventor: PARK, Jeong Je, Hwaseong-si Gyeonggi-do 18442 (KR); LEE, Jin Won, Seoul 02598 (KR); NAH, Ji Seon, Seoul 07044 (KR); PARK, Hyo Seon, Seoul 07287 (KR); KIM, Jin Ho, Seoul 08786 (KR)
(74) Representative: HGF
(86) International application number: PCT/KR2019/001010
(87) International publication number: WO 2019/160254

(56) References cited:
- WO-A1-2017/194458
- WO-A2-2017/040700
- JP-A- 2005 185 829
- JP-A- 2013 099 553
- KR-A- 20090 079 940
- KR-B1- 101 225 899
- US-A1- 2016 287 149
- US-A1- 2017 093 869

## Description

### TECHNICAL FIELD

The present disclosure relates to a method of managing biometric information and, more specifically, to a method of managing biometric information capable of preventing the use of a sensor module of which the expiration period has expired or a defective sensor module, by determining the validity of a sensor module of a continuous biometric information measuring device on the basis of the identifier, manufacturing date or production lot number out of the sensor module in sensor
usage information received from the continuous biometric information measuring device, and preventing reuse or shared use of the sensor module on the basis of user information provided in a measurement message received from the continuous biometric information measuring device.

### BACKGROUND ART

Diabetes is a chronic disease common in modern people, and about 400 million people in the world suffer from diabetes.

Diabetes is caused by the pancreas producing an entirely, or relatively, insufficient amount of insulin due to a variety of reasons, such as obesity, stress, and bad eating habits, and due to congenital or hereditary reasons, so that glucose levels are absolutely high in blood, instead of being balanced in blood.

Blood contains a certain concentration of glucose, from which tissue cells produce energy.

However, when glucose levels are higher than normal, glucose is not stored appropriately in the liver, muscles, or fat cells and excessive amounts of glucose remain in the blood.

Thus, diabetic patients commonly have higher blood glucose levels than other people. Excessive amounts of blood glucose may be discharged in urine without being absorbed by body tissues. Accordingly, since the body tissues may fail to have sufficient amounts of glucose, necessary for normal functioning, the body tissues may malfunction.

Diabetes has subtle or no subjective symptoms in the early steps of the disease. With the progression of the disease, however, the classic symptoms of diabetes, such as polydipsia, polyphagia, polyuria, weight loss, fatigue, itchy skin, and slow healing of cuts of the hands and feet, appear. Prolonged diabetes may cause complications, such as blurred vision, high blood pressure, nephropathy, palsy, periodontal diseases, muscle spasms, neuralgia, and gangrene.

In order to diagnose and manage diabetes such that complications do not arise, systematic measurement of blood glucose should be carried out in concert with systematic treatment.

A glucose monitoring device uses a method in which a user performs a blood glucose measurement once by drawing blood from a fingertip and a method in which the glucose monitoring system is attached to a user's abdomen and arm and blood glucose measurement is continuously performed.

In the case of the one-by-one blood glucose measurement method using a disposable blood glucose strip sensor, since a blood sample should be taken by directly using a needle or the like every time a blood sample is obtained, there is a disadvantage in that pain or stress is accompanied in taking a blood sample. Thus, a continuous blood glucose measurement technology using a continuous blood glucose sensor has recently been developed.

Moreover, in the case of a diabetic patient who has progressed into a serious condition, a blood glucose level needs to be more strictly managed, so a continuous blood glucose measurement system that continuously performs blood glucose measurement is used.

The continuous blood glucose measurement system includes a continuous blood glucose meter arranged on the user's skin to continuously measure the user's blood glucose, a user terminal that receives the blood glucose information from the continuous blood glucose meter and outputting the blood glucose information to the user according to a user's command, and a management server that receives and stores and registers the blood glucose information from the user terminal to provide the management information related to the continuous blood glucose meter.

Here, the continuous blood glucose meter includes a sensor module that periodically measures the user's blood glucose from a user's body fluid, a transmitter that transmits the blood glucose information measured by the sensor module to the user terminal, and the like.

Typically, the sensor module of the continuous blood glucose meter has a certain expiration date according to the measurement method such as enzymatic method/non-enzymatic method, so the user needs to measure the user's blood glucose information by attaching a sensor module that has not expired the expiration date to the skin. However, there may be cases in which a user accidentally uses a sensor module whose expiration date has elapsed, or a user intentionally uses a sensor module whose expiration date has elapsed since he/she thinks of the sensor module as being still useful.

In addition, the sensor module of the continuous blood glucose meter may cause a great health problem such as infection or the like due to the characteristics that the sensor module should be inserted into the body when reused or shared by multiple users. In fact, the sensor module of the conventional continuous blood glucose meter may often be reused or shared by multiple users.

By using the sensor module of the continuous blood glucose meter whose expiration date has expired, the user may obtain inaccurate measurements that are beyond the accuracy performance guaranteed by the manufacturer, so that the user may not receive proper blood glucose control and treatment due to inaccurate blood glucose information, or the user may be subjected to infection or the like due to reuse or shared use of the sensor module of the continuous blood glucose meter.

In the case of a conventional application running on a user terminal that stores the blood glucose information received from the continuous blood glucose meter and provides the blood glucose information to a user, there is a limitation that it cannot overcome this problem.
WO2017194458A1 discloses a skin-mountable patch device (1, 2) for use in an analyte measurement system (1, 2,3) designed for continuous in-vivo measurement of a body fluid analyte concentration, and a method for initializing the analyte measurement system.
US2017093869A1 discloses systems, devices, and methods that allow the authentication of devices within analyte monitoring systems. The analyte monitoring systems can be in vivo systems and can include a sensor control device with a sensor and accompanying circuitry, as well as a reader device for communicating with the sensor control device.
US2016287149A1 discloses a continuous glucose monitoring system and a monitoring terminal are provided, wherein the monitoring system comprises a monitoring terminal and a mobile terminal; wherein the monitoring terminal includes a portable host and probe assembly which is assembled to the host; the mobile terminal includes a second data communication unit and a User interface; the probe assembly includes two glucolase micro electrode needles, a micro processor, and two electrode terminals; the host includes a signal sampling unit, a signal processing unit, a data storage unit, and a first data communication unit.

### DISCLOSURE

### Technical Problem

Accordingly, the present disclosure has been made in consideration of the above-described problems occurring in the related art, and an objective of the present disclosure is to provide a biometric information management method that helps a user effectively use a continuous biometric information measuring device by using sensor usage information received from the continuous biometric information measuring device.

Another objective of the present disclosure is to provide a biometric information management method capable of determining the remaining use period of a sensor module on the basis of sensor usage information received from a continuous biometric information measuring device to inform the user of the replacement timing of the sensor module according to the remaining use period.

A further objective of the present disclosure is to provide a biometric information management method capable of preventing the reuse or shared use of a same sensor module by comparing user information provided in a measurement message received from a continuous biometric information measuring device with user information registered in a user terminal.

### Technical Solution

According to an aspect of the present disclosure, provided is a method of managing biometric information measured by a continuous biometric information measuring device in a user terminal. The method may include: performing a paired connection between the continuous biometric information measuring device having a sensor module and a transmitter; initializing and registering the sensor module of the continuous biometric information measuring device by receiving first sensor usage information stored in the sensor module of the continuous biometric information measuring device and storing second sensor usage information in the sensor module of the continuous biometric information measuring device; and receiving a measurement message having biometric information measured by the continuous biometric information measuring device after the initializing and registering of the sensor module and registering and storing the biometric information by mapping the biometric information to a user.

Here, the first sensor usage information may be a registered sensor usage information previously registered in a memory of the sensor module or a generated sensor usage information generated in a case in which the sensor module and the transmitter are connected, and the second sensor usage information may be user information registered in the user terminal.

Here, the registered sensor usage information may be at least one of an identifier, a manufacturing date, and a production lot of the sensor module, and the generated sensor usage information may be at least one of a use-start time of the sensor module and an identifier of the transmitter.

Preferably, the initializing and registering of the sensor module may include: receiving the identifier, the manufacturing date, or the production lot stored in the sensor module of the continuous biometric information measuring device from the continuous biometric information measuring device;
determining whether or not the sensor module has validity of use in accordance with the manufacturing date or production lot information of the sensor module; if the sensor module has the validity of use, transmitting registered user information to the continuous biometric information measuring device; and
initializing and registering a use of the sensor module in accordance with an identifier of the sensor module and the use-start time of the sensor module.

Preferably, the determining of the validity of use of the sensor module may include: determining an expiration period of the sensor module from the identifier or the manufacturing date of the sensor module and determining whether or not the expiration period of the sensor module has expired by comparing the expiration period of the sensor module with a current date, or determining whether or not the sensor module is defective by comparing the identifier or the production lot of the sensor module with a defect list received from a management server.

Preferably, the initializing and registering of the sensor module may further include: if the expiration period of the sensor module has expired or if the sensor module is defective, generating a warning message and activating and outputting the warning message to the user.

Preferably, the initializing and registering of the sensor module may further include: determining whether or not a user confirmation command for the activated warning message is input; and if the user confirmation command is input, transmitting information on an activation time of the warning message, an input time of the user confirmation command, and the identifier of the corresponding sensor module to the management server.

Preferably, the storing of the biometric information may include: comparing first user information provided in the measurement message with registered user information; determining whether or not second user information identical to the first user information is present in the registered user information; and registering and storing the biometric information provided in the measurement message by mapping the biometric information to the second user information identical to the first user information.

Preferably, the registering and storing of the biometric information may further include: if no user information identical to the first user information is present in the registered user information, generating a user confirmation message and activating and outputting the user confirmation message to the user.

Preferably, the biometric information management method may further include calculating a remaining use period in accordance with use-start time information and a usage period of the sensor module.

Preferably, the biometric information management method may further include: when a remaining use period confirmation command is input, calculating a remaining use period in accordance with an actual usage period and the usage period of the sensor module, the actual usage period extending from the use-start time to a time at which the remaining use period confirmation command is input, and outputting information on the remaining use period to the user.

Here, the biometric information management method may further include: if the remaining use period is less than a set threshold, generating a replacement notification message, and activating and outputting the generated replacement notification message to the user.

### Advantageous Effects

The biometric information measurement method according to the present disclosure has the following effects.

First, the biological information management method may determine the validity of the sensor module of the continuous biological information measuring device on the basis of the manufacturing date or production lot information of the sensor usage information received from the continuous biological information measuring device, thereby preventing reuse of the sensor module whose expiration period has expired, or of defective sensor module.

Second, the biometric information management method may determine the remaining use period of the sensor module on the basis of the sensor usage information received from the continuous biometric information measuring device, thereby informing the user of the replacement time of the sensor module according to the remaining use period.

Third, the biometric information measurement method may compare the user information provided in the measurement message received from the continuous biometric information measuring device with the user information registered in the user terminal and maps the received biometric information to corresponding user information for registration management, thereby preventing reuse or shared use of the same sensor module.

Fourth, the biometric information measurement method may compare user information provided in the measurement message received from the continuous biometric information measuring device with user information registered in the user terminal, and if the received user biometric information is different from the user information registered in the user terminal, prevent the biometric information from being displayed, thereby preventing the leakage of important biometric information.

### DESCRIPTION OF DRAWINGS

FIG. 1 is a diagram illustrating a continuous biometric Information disclosure; management system according to the present
FIG. 2 is a diagram illustrating an example of a continuous biometric information measuring device 100 according to the present disclosure;
FIG. 3 is a functional block diagram illustrating a user terminal for managing biometric information according to the present disclosure;
FIG. 4 is a functional block diagram illustrating an initializer and register according to an embodiment of the present disclosure;
FIG. 5 is a functional block diagram illustrating an information manager according to an embodiment of the present disclosure;
FIG. 6 is a flowchart illustrating a method of managing biometric information using a user termin according to the present disclosure;
FIG. 7 is a flowchart illustrating a step of initializing and registering a sensor module according to an embodiment of the present disclosure;
FIG. 8 illustrates an example of a warning message and a replacement message according to the present disclosure; and
FIG. 9 is a flow chart illustrating a step of informing a user of the remaining use period of the sensor module according to an embodiment of the present disclosure.

### MODE FOR INVENTION

The technical or scientific terms used herein are for the purpose of describing particular exemplary embodiments only and are not intended to be limiting of the present disclosure. In addition, the technical or scientific terms used herein should be understood as having the same meaning as commonly understood by a person having ordinary knowledge in the art to which the present disclosure relates, and should not be interpreted in an overly broad or narrow manner, unless expressly so defined herein. When any technical terms used herein fail in correctly representing the idea of the present disclosure, they should be substituted with technical terms correctly understandable by a person having ordinary knowledge in the art to which the present disclosure relates.

Descriptions of components in the singular form used herein are intended to include descriptions of components in the plural form, unless explicitly described to the contrary. It will be understood that the terms "comprise," "include," and any variations thereof used herein shall not be interpreted as essentially including all components or steps described herein, and are intended to cover non-exclusive inclusions unless explicitly described to the contrary.

In addition, the accompanying drawings shall be interpreted as being provided for a better understanding, while not being limitative, of the principle of the present disclosure.

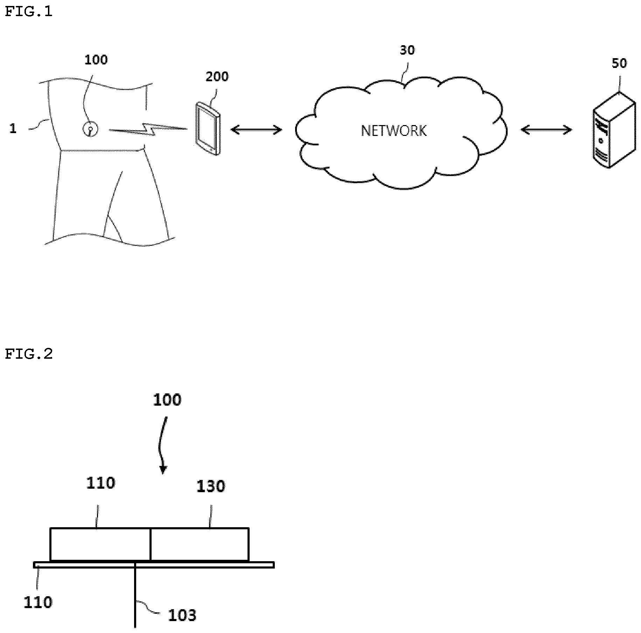

FIG. 1 is a diagram illustrating a continuous biometric information management system according to the present disclosure.

Referring specifically to FIG. 1, a continuous biometric information measuring device 100 is attached to the skin of the user 1. The continuous biometric information measuring device 100 periodically extracts the body fluid of the user 1 to measure the user's biometric information, for example, blood glucose, using the extracted body fluid.

A user terminal 200 is connected to the continuous biometric information measuring device 100 via short-range communication, for example, Bluetooth communication^{®}, infrared communication, and the like. The continuous biometric information measuring device 100 and the user terminal 200 transmit and receive data to and from each other via short- range communication. That is, when the continuous biometric information measuring device 100 and the user terminal 200 are located in a short-range communication range of each other, the continuous biometric information measuring device 100 transmits measured biometric information of the user 1 to the user terminal 200.

Here, the user terminal 200 is a device capable of storing, or outputting to the user, the user's biometric information received from the continuous biometric information measuring device 100 so that the user may review the information. The user terminal may execute an application for managing the biometric information. As an example of such a user terminal 200, a smart phone, a notebook PC, a PDA, a dedicated terminal for managing biometric information, and the like may be used.

A management server 50 is connected to the user terminal 200 via the network 30. The user terminal 200 transmits the biometric information of the user 1 received from the continuous biometric information measuring device 100 to the management server 50 or the sensor usage information of the continuous biometric information measuring device 100 to the management server 50. Meanwhile, the user terminal 200 may receive product information for each production lot or expiration date according to the identifier of the continuous biometric information measuring device from the management server 50.

Preferably, the user terminal 200 identifies a user on the basis of the user information, and registers and stores the received biometric information by mapping the received biometric information to the identified user. Meanwhile, the user terminal 200 transmits the registered biometric information mapped to the user or sensor usage information to the management server 50, together with the user information.

The continuous biometric information measuring device according to the present disclosure includes a sensor module that extracts a user's body fluid to measure user's biometric information, and a transmitter that transmits the measured biometric information to the user terminal. The sensor module includes a separate memory storing sensor usage information to allow the user to use an effective continuous biometric information measuring device by using the sensor usage information, or to prevent reuse or shared use by multiple users of the continuous biometric information measuring device.

FIG. 2 is an example diagram illustrating a continuous biometric information measuring device 100 according to the present disclosure.

The continuous biometric information measuring device 100 according to the present disclosure includes a sensor module 110 attached to the user's skin to extract the user's body fluid and periodically measure the user's biometric information from the body fluid, and a transmitter 130 detachably connected to the sensor module 110 to transmit the biometric information measured by the sensor module 110 to the user terminal. Here, the sensor module 110 and the transmitter 130 are manufactured as separate modules, and the sensor module 110 and the transmitter 130 are coupled together in a detachable manner.

A connection terminal (not shown) is formed on one side of the sensor module 110 so that the sensor module 110 and the transmitter 130 are connected to each other through the connection terminal. The biometric information measured by the sensor module 110 may be transmitted to the transmitter 130 through the connection terminal.

The sensor module 110 and the transmitter 130 are disposed on the user's skin through an attachment pad 101, formed to be attached to a part of the body, for example, the skin of the belly or flank. The attachment methods of the attachment pad 101 to the body may be set in various manners, such as an attachment method using a separate fixing band or an attachment method in which an adhesive is applied to the edge portion so that the adhesive edge portion is attached to the skin.

At this time, a body fluid extraction tube 103 may be arranged to be partially inserted into the body while the attachment pad 101 is attached to the body. For example, the body fluid extraction tube is disposed such that an end thereof protrudes from a body's attachment surface of the attachment pad 101. According to this structure, when the attachment pad 101 is attached to the body surface, the body fluid extraction tube 103 attached to the attachment pad 101 is disposed such that the end portion thereof is inserted into the body.

Here, the body fluid may include either cell interstitial fluid or blood, and the blood conceptually includes any one of capillary blood, venous blood, and arterial blood. In addition, the body fluid extraction tube 103 may be provided in various forms capable of extracting body fluids from the body. Such forms including cannula, microdialysis tubing, or the like are collectively referred to as a body fluid extraction tube.

When the attachment pad 101 is attached to the body in this manner, since the body fluid extraction tube 103 is maintained in the body, the user's body fluid, extracted through the body fluid extraction tube 103 in this state and fed through a connection tube, is measured with respect to biometric information, for example, blood glucose concentration, by the sensor module 110.

The sensor module 110 measures the blood glucose concentration using the body fluid. The blood glucose concentration may be measured using various types of sensors, such as a measuring sensor used in a conventional blood collection-type blood glucose meter. In addition, since existing various blood glucose sensors may be applied, a detailed description thereof will be omitted.

The sensor module 110 is provided with a separate storage (not shown), which registers and stores sensor usage information to manage the effective use of the sensor module 110, prevents reuse or shared use of the sensor module, or informs the user of the replacement timing of the sensor module. The sensor usage information registered and stored in the storage includes sensor usage information previously registered in a sensor module 110 at shipping time, sensor usage information generated when the sensor module 110 is connected to the transmitter, and sensor usage information received from the user terminal via the transmitter 130.

Here, the registered sensor usage information is an identifier of the sensor module, a manufacturing date, a production lot, an expiration date, a predetermined usage period or a predetermined number of uses, and the generated sensor usage information is a start time when the sensor module is used, and an identifier of a connected transmitter, and the received sensor usage information is the user information received from the user terminal.

The production lot information is provided for the following reason. Since the meter or measuring element of the sensor module of the blood glucose meter is a complex of several substances, such as a metal, an enzyme, a film, or the like, and the production conditions thereof, such as temperature, humidity, and user's proficiency, are also different, it is theoretically impossible to produce sensor modules with the same performance and properties. Thus, mass-produced sensor modules may be divided into lots based on production lot information so that the deviations of the sensor modules that appear according to the production lot information may be determined, thereby accurately measuring biometric information.

Here, the user information may be user information registered in a biometric information management application executed in a user terminal. For example, the user information may include a unique identifier such as a user ID, password, date of birth, social security number, or the like.

The sensor usage information measured by or stored in the sensor module 110 is transmitted to an accessed user terminal in a short-range communication range (when the short-range communication is Bluetooth communication, a paired user terminal) through the transmitter 130. The biometric information of is transmitted through the transmitter 130 to the connected user terminal located in the short range communication range.

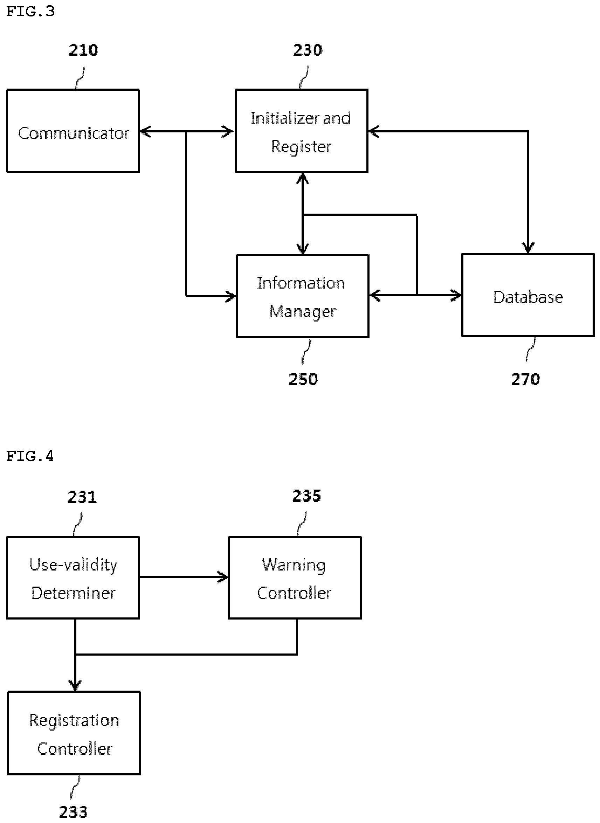

FIG. 3 is a functional block diagram illustrating a user terminal for managing biometric information according to the present disclosure.

Referring to FIG. 3 in more detail, a communicator 210 is connected to the continuous biometric information measuring device to transmit/receive data to/from the continuous biometric information measuring device or to a management server to transmit/receive data to/from the management server. The communicator 210 may perform short-range communication for transmitting and receiving data with the continuous biometric information measuring device and Internet communication for transmitting and receiving data with the management server.

Preferably, the communicator 210 may use infrared communication, Bluetooth communication, or the like to transmit and receive data in a short-range communication method with the continuous biometric information measuring device. When employing Bluetooth communication, the communicator performs a paired connection to the continuous biometric information measuring device.

When the communication connection with the continuous biometric information measuring device is completed, an initializer and register 130 initializes and registers the sensor module by receiving the registered sensor usage information such as the identifier, the manufacturing date, and the production lot stored in the sensor module of the continuous biometric information measuring device or transmitting the sensor usage information, such as user information, to the biometric information measuring device, and storing the received sensor usage information and information on the start time of the sensor module in a database 270.

The initializer and register 230 may determine the validity of use of the sensor module by determining whether or not the expiration date of the sensor module has expired on the basis of the manufacturing date of the received sensor usage information or determining whether or not the sensor module is defective on the basis of lot information of the sensor usage information.

Meanwhile, when the sensor module is initialized and registered, an information manager 250 periodically receives biometric information from the continuous biometric information measuring device through the communicator 210, compares the user information of the received biometric information with the user information registered in the user terminal so that the received biometric information is mapped to the registered user information, and registers and stores the received biometric information in the database270. The information manager 250 periodically transmits the biometric information registered and stored in the database 270 to the management server at the request of the management server.

FIG. 4 is a functional block diagram illustrating an initializer and register according to an embodiment of the present disclosure.

Referring more specifically to FIG. 4, a use-validity determiner 231 determines the validity of use of the sensor module, **i.e.** whether or not the sensor module is effectively used, on the basis of the identifier, manufacturing date or production lot information of the sensor module in the sensor usage information received from the continuous biometric information measuring device. That is, the use-validity determiner 231 determines whether or not the expiration date of a target sensor module has expired on the basis of the identifier or the manufacturing date of the sensor module in the sensor usage information, or determines whether or not a target sensor module is defective on the basis of the production lot information in the sensor usage information.

Preferably, when receiving the sensor module identifier from the continuous biometric information measuring device, the use-validity determiner 231 transmits the sensor module identifier to the management server to receive the expiration date corresponding to the sensor module identifier. Thus, the use-validity determiner may determine whether or not the expiration date of the sensor module has expired on the basis of the current date, the manufacturing date, and expiration date of the sensor module.

Preferably, when receiving the production lot information from the continuous biometric information measuring device, the use-validity determiner 231 transmits the production lot information to the management server to receive the information about defective product corresponding to the production lot information. Thus, the use-validity determiner may determine whether or not the sensor module is defective on the basis of the defective product information for production lots.

When the use-validity determiner 231 determines that the sensor module is effectively used, a registration controller 233 registers and stores the registered sensor usage information such as the identifier, manufacturing date, and production lot of the sensor module in the received information in the database, or registers and stores the generated sensor usage information, such as the start time of the sensor module or the identifier of the transmitter, in the database. On the other hand, the registration controller 233 transmits the user information registered in the database to the continuous biometric information measuring device through the communicator when the corresponding sensor module is effectively used.

Here, the registration controller 233 may use the connection time with the continuous biometric information measuring device as the start time of the sensor module or may use the connection time between the sensor module and the transmitter, which is received from the continuous biometric information measuring device, as the start time of the sensor module.

However, when the use-validity determiner 231 determines that the corresponding sensor module is not effectively used, that is, it is determined that the corresponding sensor module has elapsed expiration date or is defective, a warning controller 235 generates a warning message and activates and outputs the generated warning message to the user terminal.

When receiving a user's confirmation command for the activated warning message, the warning controller 235 registers and stores information on the time when the warning message is activated and output and the time when the confirmation command is received in the database 270, or transmits the same to the management server.

In an embodiment, when receiving the user's confirmation command for the warning message, the warning controller 235 controls the registration controller 233 to reject the initializing and registering of the sensor module, thereby disabling the user from using the invalid sensor module.

In another embodiment, when receiving the user's confirmation command for the warning message, the warning controller 235 may periodically generate and output, to the user, a warning message for informing the user of the replacement of the sensor module while controlling the registration controller 233 to initialize and register the sensor module.

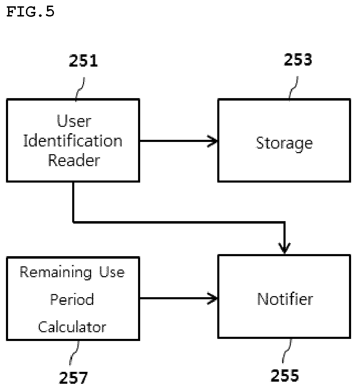

FIG. 5 is a functional block diagram illustrating an information manager according to an embodiment of the present disclosure.

Referring more specifically to FIG. 5, when receiving a measurement message from the continuous biometric information measuring device, a user identification reader 251 identifies the user by comparing the user information provided in the measurement message with the user information registered in the user terminal and determining the presence of user information identical to the user information in the measurement message in the registered user information. Upon initializing and registering of the sensor module, the registration controller transmits the registered user information to the sensor module, and the continuous biometric information measuring device has or uses the user information received when the measurement message is generated.

If the user information identical to the user information is present, a storage 253 registers the biometric information of the received measurement message in the database by mapping the biometric information to the identical user information.

However, if the user information identical to the user information is not present, a notifier 255 generates a user confirmation message and activates and outputs the generated user confirmation message to the user terminal, and the storage 253 does not perform the registering and storing of the biometric information of the received measurement message in the database.

On the other hand, when receiving a remaining use period confirmation command through the user terminal, a remaining use period calculator 257 calculates remaining use period of the sensor module from usage-start time of the sensor module, actual usage time of the sensor module until the remaining use period confirmation command is input, and the expiration date of the sensor module, and activates and outputs the calculated remaining use period information to the user terminal.

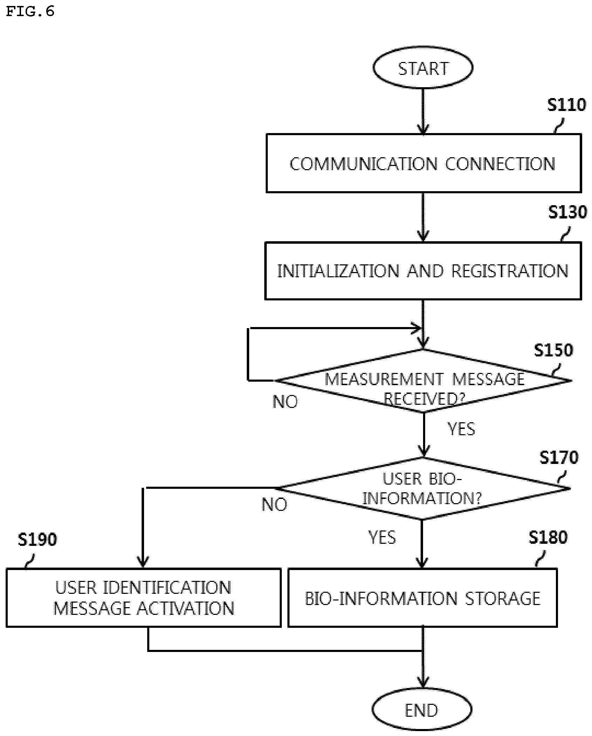

FIG. 6 is a flowchart illustrating a method of managing biometric information using a user terminal according to the present disclosure.

Referring more specifically to FIG. 6, the user terminal performs a communication connection with the continuous biometric information measuring device in Sll0. Here, the user terminal and the continuous biometric information measuring device perform infrared communication or Bluetooth communication. Since such communication connection is a well-known technique, a detailed description thereof will be omitted.

The user terminal initializes and registers the sensor module of the continuous biometric information measuring device to the user terminal by receiving the first sensor usage information from the continuous biometric information measuring device or by transmitting the second sensor usage information to the continuous biometric information measuring device in S130. Here, the first sensor usage information is sensor usage information previously registered in the memory of the sensor module or sensor usage information generated when the sensor module and the transmitter are connected, and the second sensor usage information is user information registered in the user terminal. The registered sensor usage information is at least one of the identifier, manufacturing date, and production lot of the sensor module, and the generated sensor usage information is at least one of the usage-start time of the sensor module and the identifier of the transmitter.

When the sensor module is initialized and registered in the user terminal, the user terminal determines the validity of use of the sensor module on the basis of the identifier, the manufacturing date, or production lot information of the sensor module in the received first sensor usage information. When it is determined that the use of the sensor module is valid, the sensor module may be initialized and registered in the user terminal.

When the initializing and registering of the sensor module is completed, the user terminal periodically receives a measurement message with biometric information from the continuous biometric information measuring device in S150, and determines whether or not registered user information identical to the received user information is present in the user information log-in registered the user terminal by comparing the user information in the received measurement message and the log-in registered user information in S170. In the initializing and registering of the sensor module, the user information using the sensor module is transmitted to the sensor module, and the continuous biometric information measuring device generates the measurement message with the user information or the measurement message encrypted with the user information.

When the registered user information identical to the received user information is present, the received biometric information is mapped to the identical user information and stored in the database in S180.

However, when there is no registered user information identical to the received user information, the user confirmation message is activated and output to the user terminal in S190.

With the provision of user information in the measurement message or encryption of the measurement message with user information, when receiving the measurement message, the user terminal may manage the biometric information by comparing the user information in the measurement message with the user information input to the application of the user terminal and mapping the user information to the identical user. This may prevent third parties from being able to view the user's biometric information, or prevent different users from co-using or reusing the same sensor module.

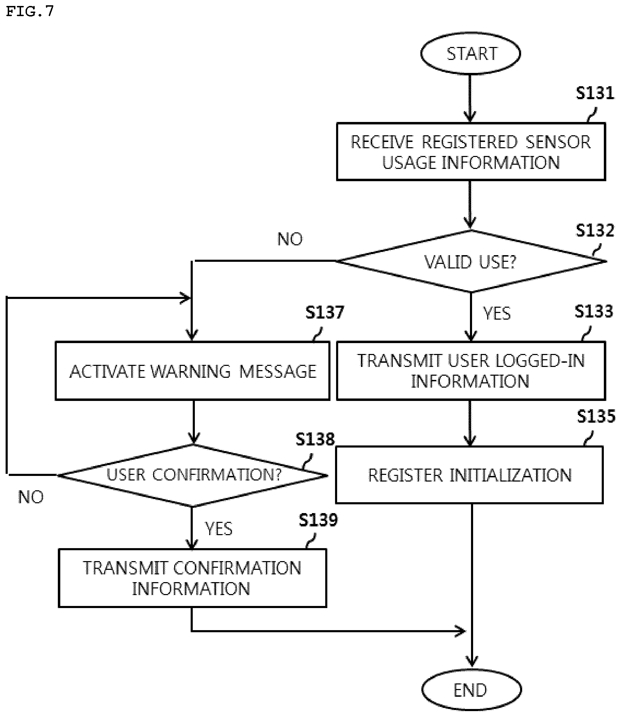

FIG. 7 is a flowchart illustrating a step of initializing and registering a sensor module according to an embodiment of the present disclosure.

Referring more specifically to FIG. 7, registered sensor usage information, such as the identifier, the manufacturing date, the production lot, and the like of the sensor module, is received from the continuous biometric information measuring device in S131.

On the basis of the received registered sensor usage information, it is determined whether or not a target sensor module to be used by the user may be validly used in S132. Here, in order to determine the validity of use of the sensor module, it is determined whether or not an expiration date of the sensor module has expired on the basis of the identifier or the manufacturing date of the sensor module in the registered sensor usage information, or whether or not the sensor module is defective on the basis of production lot information in the registered sensor usage information.

When the sensor module to be used by the user is a product that may be validly used, the user information registered in the user terminal is extracted and the extracted user information is transmitted to the continuous biometric information measuring device to register and store the user information in the memory of the sensor module in S133.

The sensor usage information generated by the continuous biometric information measuring device is registered and stored together with the registered sensor usage information of the sensor module, thereby completing the initializing and registering of the sensor module in S135. Here, the generated sensor usage information is the use-start time of the sensor module or the identifier of the transmitter. As the use-start time of the sensor module, the connection time between the sensor module and the transmitter or between the continuous biometric information measuring device and the user terminal may be used.

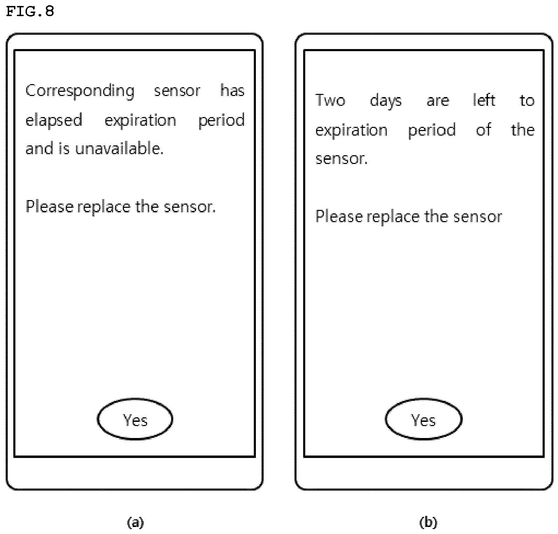

Meanwhile, when a target sensor module to be used by the user is a product that cannot be validly used, a warning message is generated and the generated warning message is activated and output through a display of the user terminal in S137. FIG. 8 (a) illustrates an example of a warning message.

As illustrated in FIG. 8 (a), the warning message indicates that the corresponding sensor module cannot be used due to elapsed expiration date or cannot be used due to defectiveness.

When a user confirmation command is received from the user terminal in response to the activated warning message in S138, time information when the warning message is activated and output to the user and the information about the time when the user confirmation command is received are stored and registered in the database, or are transmitted to the management server in S139. By holding the time information when the warning message is activated and output to the user and the information about the time when the user confirmation command is received, the responsibility of the sensor module manufacturer caused by the future use of the invalid sensor module by the user may be reduced when the user uses the invalid sensor module after being advised not to do so.

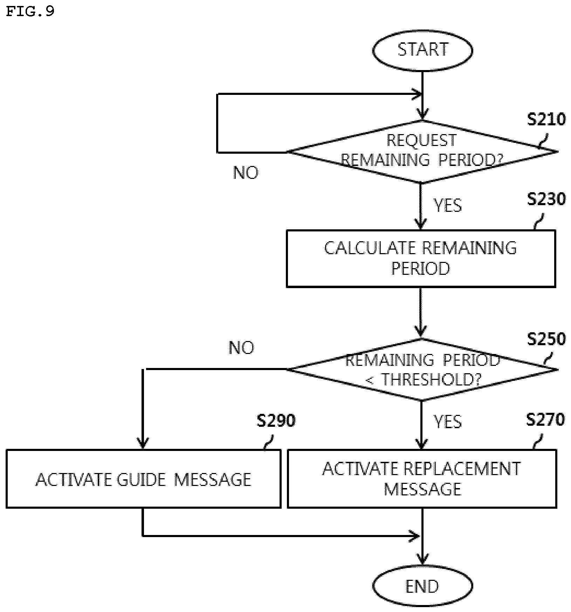

FIG. 9 is a flow chart illustrating a step of informing a user of the remaining use period of the sensor module according to an embodiment of the present disclosure.

When a user command for requesting the remaining use period of the sensor module is input through the user terminal S210, the actual usage period of the sensor module is calculated from the input time of the user command for requesting the remaining use period and the use-start time of the sensor module, and the remaining use period of the sensor module is calculated from the expiration period and the actual usage period of the sensor module S230.

The calculated remaining use period is compared with a set threshold expiration period S250, and when the remaining use period is less than the threshold expiration period, a replacement message is generated and the generated replacement message is activated and output through the display of the user terminal S270. FIG. 8 (b) shows an example of the replacement message, wherein the replacement message may include the remaining use period of the sensor module being used by the user and the replacement request for the replacement of the sensor module being used.

On the other hand, when the remaining use period exceeds the threshold period, a guide message having a remaining use period is generated and the generated guide message is activated and output to the display of the user terminal S290.

While the present disclosure has been described with reference to certain exemplary embodiments shown in the drawings, these embodiments are illustrative only. Rather, it will be understood by a person having ordinary knowledge in the art that various modifications and equivalent other embodiments may be made therefrom. Therefore, the true scope of the invention is defined by the appended claims.

## Claims

1. A method of managing biometric information measured by a continuous biometric information measuring device (100) in a user terminal (200), the method comprising:
performing a paired connection between the continuous biometric information measuring device (100) having a sensor module (110) and a transmitter (130), and the user terminal (200) through the transmitter (130);
initializing and registering the sensor module (110) of the continuous biometric information measuring device by receiving first sensor usage information stored in the sensor module (110) of the continuous biometric information measuring device (100) and storing second sensor usage information in the sensor module (110) of the continuous biometric information measuring device (100); and
receiving a measurement message having biometric information measured by the continuous biometric information measuring device (100) after the step of initializing and registering of the sensor module (110) and registering and storing the biometric information in the user terminal (200) by mapping the biometric information to a user,
wherein the initializing and registration of the sensor module (110) further comprises:
determining whether the sensor module (110) is valid for use based on the first sensor usage information;
in case of determining that the sensor module (110) is not valid for use, generating a warning message and activating and outputting the warning message to the user terminal (200);
**characterized in that** in response to the warning message on the user terminal (200), receiving a user confirmation through the user terminal (200) and transmitting the information including an activation time of the warning message and an input time of the user confirmation command to a server (50).

2. The method according to claim 1, wherein the first sensor usage information comprises sensor usage information previously registered in a memory of the sensor module (110) or sensor usage information generated in a case in which the sensor module (110) and the transmitter (130) are connected, and
the second sensor usage information is user information registered in the user terminal (200).

3. The method according to claim 2, wherein the registered sensor usage information is at least one of an identifier, a manufacturing date, and a production lot of the sensor module (110), and
the generated sensor usage information is at least one of a use-start time of the sensor module (110) and an identifier of the transmitter (130).

4. The method according to claim 3, wherein the step of determining whether the sensor module (110) is valid for use is based on the manufacturing date or production lot information of the sensor module (110);
in case of determining that the sensor module (110) is valid for use, transmitting registered user information to the continuous biometric information measuring device (100); and
initializing and registering a use of the sensor module (110) in accordance with an identifier of the sensor module and the use-start time of the sensor module.

5. The method according to claim 4, wherein the step of determining whether the sensor module (110) is valid for use further comprises:
determining an expiration period of the sensor module (110) from the identifier or the manufacturing date of the sensor module and determining whether the expiration period of the sensor module has expired by comparing the expiration period of the sensor module (110) with a current date, or
determining whether the sensor module (110) is defective by comparing the identifier or the production lot of the sensor module (110) with a defect list received from the server (50).

6. The method according to claim 5, wherein the step of initializing and registering of the sensor module (110) further comprises:
in case of determining that the expiration period of the sensor module (110) has expired or that the sensor module (110) is defective, generating a warning message and activating and outputting the warning message to the user terminal (200).

7. The method according to claim 6, wherein the step of initializing and registering of the sensor module (110) further comprises:
in case of determining that a user confirmation command for the activated warning message is input, transmitting information on an activation time of the warning message, an input time of the user confirmation command, and the identifier of the corresponding sensor module (110) to the management server (50).

8. The method according to claim 3, wherein the storing of the biometric information comprises:
comparing the first user information provided in the measurement message with the registered user information;
determining whether the second user information identical to the first user information is present in the registered user information; and
registering and storing the biometric information provided in the measurement message by mapping the biometric information to the second user information identical to the first user information.

9. The method according to claim 8, wherein the step of registering and storing the biometric information further comprises:
in case of determining that the second user information identical to the first user information is not present in the registered user information, generating a user confirmation message and activating and outputting the user confirmation message to the user terminal (200).

10. The method according to claim 3, further comprising calculating a remaining use period in accordance with use-start time information and a usage period of the sensor module (110).

11. The method according to claim 10, further comprising: in case of a remaining use period confirmation command being input, calculating a remaining use period in accordance with an actual usage period and the usage period of the sensor module (110), the actual usage period extending from the use-start time to a time at which the remaining use period confirmation command is input, and outputting information on the remaining use period to the user.

12. The method according to claim 10, further comprising: if the remaining use period is less than a set threshold, generating a replacement notification message and activating and outputting the generated replacement notification message to the user.

## Patentansprüche

1. Verfahren zum Verwalten biometrischer Informationen, die von einer kontinuierlichen Messvorrichtung (100) für biometrische Informationen in einem Benutzerendgerät (200) gemessen werden, wobei das Verfahren Folgendes umfasst:
Durchführen einer gekoppelten Verbindung zwischen der kontinuierlichen Messvorrichtung (100) für biometrische Informationen, die ein Sensormodul (110) und einen Sender (130) aufweist, und dem Benutzerendgerät (200) durch den Sender (130);
Initialisieren und Registrieren des Sensormoduls (110) der kontinuierlichen Messvorrichtung für biometrische Informationen, indem erste Sensornutzungsinformationen, die in dem Sensormodul (110) der kontinuierlichen Messvorrichtung (100) für biometrische Informationen gespeichert sind, empfangen werden und zweite Sensornutzungsinformationen in dem Sensormodul (110) der kontinuierlichen Messvorrichtung (100) für biometrische Informationen gespeichert werden; und
Empfangen einer Messmeldung mit biometrischen Informationen, die von der kontinuierlichen Messvorrichtung (100) für biometrische Informationen gemessen werden, nach dem Schritt des Initialisierens und Registrierens des Sensormoduls (110) und Registrierens und Speicherns der biometrischen Informationen in dem Benutzerendgerät (200), indem die biometrischen Informationen einem Benutzer zugeordnet werden,
wobei das Initialisieren und Registrieren des Sensormoduls (110) ferner Folgendes umfasst:
Bestimmen, ob das Sensormodul (110) zur Verwendung gültig ist, basierend auf den ersten Sensornutzungsinformationen;
im Falle des Bestimmens, dass das Sensormodul (110) nicht zu Verwendung gültig ist, Erzeugen einer Warnmeldung und Aktivieren und Ausgeben der Warnmeldung an das Benutzerendgerät (200); **dadurch gekennzeichnet, dass**
als Reaktion auf die Warnmeldung auf dem Benutzerendgerät (200) Empfangen einer Benutzerbestätigung durch das Benutzerendgerät (200) und Senden der Informationen, die eine Aktivierungszeit der Warnmeldung und eine Eingabezeit des Benutzerbestätigungsbefehls beinhalten, an einen Server (50).

2. Verfahren nach Anspruch 1, wobei die ersten Sensornutzungsinformationen Sensornutzungsinformationen umfassen, die zuvor in einem Speicher des Sensormoduls (110) registriert wurden, oder Sensornutzungsinformationen, die in einem Fall erzeugt wurden, in dem das Sensormodul (110) und der Sender (130) verbunden sind, und
die zweiten Sensornutzungsinformationen Benutzerinformationen sind, die in dem Benutzerendgerät (200) registriert sind.

3. Verfahren nach Anspruch 2, wobei die registrierten Sensornutzungsinformationen mindestens eines von einer Kennung, einem Herstellungsdatum und einer Produktionscharge des Sensormoduls (110) sind und
die erzeugten Sensornutzungsinformationen mindestens eines von einer Verwendungsstartzeit des Sensormoduls (110) und einer Kennung des Senders (130) sind.

4. Verfahren nach Anspruch 3, wobei der Schritt des Bestimmens, ob das Sensormodul (110) zur Verwendung gültig ist, auf dem Herstellungsdatum oder den Produktionschargeninformationen des Sensormoduls (110) basiert;
im Falle des Bestimmens, dass das Sensormodul (110) zu Verwendung gültig ist, Senden registrierter Benutzerinformationen an die kontinuierliche Messvorrichtung (100) für biometrische Informationen; und
Initialisieren und Registrieren einer Verwendung des Sensormoduls (110) gemäß einer Kennung des Sensormoduls und der Verwendungsstartzeit des Sensormoduls.

5. Verfahren nach Anspruch 4, wobei der Schritt des Bestimmens, ob das Sensormodul (110) zur Verwendung gültig ist, ferner Folgendes umfasst:
Bestimmen einer Ablauffrist des Sensormoduls (110) aus der Kennung oder dem Herstellungsdatum des Sensormoduls und Bestimmen, ob die Ablauffrist des Sensormoduls abgelaufen ist, indem die Ablauffrist des Sensormoduls (110) mit einem aktuellen Datum verglichen wird, oder
Bestimmen, ob das Sensormodul (110) defekt ist, indem die Kennung oder die Produktionscharge des Sensormoduls (110) mit einer Fehlerliste, die von dem Server (50) empfangen wurde, verglichen wird.

6. Verfahren nach Anspruch 5, wobei der Schritt des Initialisierens und Registrierens des Sensormoduls (110) ferner Folgendes umfasst:
im Falle des Bestimmens, dass die Ablauffrist des Sensormoduls (110) abgelaufen ist oder dass das Sensormodul (110) defekt ist, Erzeugen einer Warnmeldung und Aktivieren und Ausgeben der Warnmeldung an das Benutzerendgerät (200).

7. Verfahren nach Anspruch 6, wobei der Schritt des Initialisierens und Registrierens des Sensormoduls (110) ferner Folgendes umfasst:
im Falle des Bestimmens, dass ein Benutzerbestätigungsbefehl für die aktivierte Warnmeldung eingegeben wird, Senden von Informationen über eine Aktivierungszeit der Warnmeldung, einer Eingabezeit des Benutzerbestätigungsbefehls und der Kennung des entsprechenden Sensormoduls (110) an den Verwaltungsserver (50).

8. Verfahren nach Anspruch 3, wobei das Speichern der biometrischen Informationen Folgendes umfasst:
Vergleichen der ersten Benutzerinformationen, die in der Messmeldung bereitgestellt werden, mit den registrierten Benutzerinformationen;
Bestimmen, ob die zweiten Benutzerinformationen, die mit den ersten Benutzerinformationen identisch sind, in den registrierten Benutzerinformationen vorhanden sind; und
Registrieren und Speichern der in der Messmeldung bereitgestellten biometrischen Informationen, indem die biometrischen Informationen den zweiten Benutzerinformationen, die mit den ersten Benutzerinformationen identisch sind, zugeordnet werden.

9. Verfahren nach Anspruch 8, wobei der Schritt des Registrierens und Speicherns der biometrischen Informationen ferner Folgendes umfasst:
im Falle des Bestimmens, dass die zweiten Benutzerinformationen, die mit den ersten Benutzerinformationen identisch sind, nicht in den registrierten Benutzerinformationen vorhanden sind, Erzeugen einer Benutzerbestätigungsmeldung und Aktivieren und Ausgeben der Benutzerbestätigungsmeldung an das Benutzerendgerät (200) .

10. Verfahren nach Anspruch 3, ferner umfassend das Berechnen einer verbleibenden Verwendungsdauer gemäß Verwendungsstartzeitinformationen und einer Nutzungsdauer des Sensormoduls (110).

11. Verfahren nach Anspruch 10, ferner umfassend: in dem Fall, dass ein Bestätigungsbefehl für eine verbleibende Verwendungsdauer eingegeben wird, Berechnen einer verbleibenden Verwendungsdauer gemäß einer tatsächlichen Nutzungsdauer und der Nutzungsdauer des Sensormoduls (110), wobei sich die tatsächliche Nutzungsdauer von der Verwendungsstartzeit bis zu einem Zeitpunkt erstreckt, zu dem der Bestätigungsbefehl für die verbleibende Verwendungsdauer eingegeben wird, und Ausgeben von Informationen über die verbleibende Verwendungsdauer an den Benutzer.

12. Verfahren nach Anspruch 10, ferner umfassend: wenn die verbleibende Verwendungsdauer kleiner als ein festgelegter Schwellenwert ist, Erzeugen einer Ersatzbenachrichtigungsmeldung und Aktivieren und Ausgeben der erzeugten Ersatzbenachrichtigungsmeldung an den Benutzer.

## Revendications

1. Procédé de gestion d'informations biométriques mesurées par un dispositif de mesure d'informations biométriques en continu (100) dans un terminal utilisateur (200), le procédé comprenant :
la réalisation d'une connexion appariée entre le dispositif de mesure d'informations biométriques en continu (100) comportant un module de capteur (110) et un émetteur (130), et le terminal utilisateur (200) par l'intermédiaire de l'émetteur (130) ;
l'initialisation et l'enregistrement du module de capteur (110) du dispositif de mesure d'informations biométriques en continu en recevant des premières informations d'utilisation de capteur stockées dans le module de capteur (110) du dispositif de mesure d'informations biométriques en continu (100) et en stockant des secondes informations d'utilisation de capteur dans le module de capteur (110) du dispositif de mesure d'informations biométriques en continu (100) ; et
la réception d'un message de mesure comportant des informations biométriques mesurées par le dispositif de mesure d'informations biométriques en continu (100) après l'étape d'initialisation et d'enregistrement du module de capteur (110) et d'enregistrement et de stockage des informations biométriques dans le terminal utilisateur (200) en mettant en correspondance les informations biométriques avec un utilisateur,
ladite initialisation et ledit enregistrement du module de capteur (110) comprenant en outre :
la détermination pour savoir si le module de capteur (110) est valide pour une utilisation sur la base des premières informations d'utilisation de capteur ;
en cas de détermination que le module de capteur (110) n'est pas valide pour une utilisation, la génération d'un message d'avertissement et l'activation et l'émission du message d'avertissement à destination du terminal utilisateur (200) ;
**caractérisé en ce que**, en réponse au message d'avertissement sur le terminal utilisateur (200), la réception d'une confirmation d'utilisateur par l'intermédiaire du terminal utilisateur (200) et la transmission des informations comprenant un temps d'activation du message d'avertissement et un temps d'entrée de la commande de confirmation d'utilisateur à un serveur (50).

2. Procédé selon la revendication 1, lesdites premières informations d'utilisation de capteur comprenant des informations d'utilisation de capteur précédemment enregistrées dans une mémoire du module de capteur (110) ou des informations d'utilisation de capteur générées dans un cas dans lequel le module de capteur (110) et l'émetteur (130) sont connectés, et
lesdites secondes informations d'utilisation de capteur étant des informations d'utilisateur enregistrées dans le terminal utilisateur (200).

3. Procédé selon la revendication 2, lesdites informations d'utilisation de capteur enregistrées étant au moins une information parmi un identifiant, une date de fabrication et un lot de production du module de capteur (110), et
lesdites informations d'utilisation de capteur générées étant au moins une information parmi une heure de début d'utilisation du module de capteur (110) et un identifiant de l'émetteur (130).

4. Procédé selon la revendication 3, ladite étape de détermination pour savoir si le module de capteur (110) est valide pour une utilisation étant basée sur les informations de date de fabrication ou de lot de production du module de capteur (110) ;
en cas de détermination que le module de capteur (110) est valide pour une utilisation, la transmission d'informations d'utilisateur enregistrées au dispositif de mesure d'informations biométriques en continu (100) ; et
l'initialisation et l'enregistrement d'une utilisation du module de capteur (110) conformément à un identifiant du module de capteur et à l'heure de début d'utilisation du module de capteur.

5. Procédé selon la revendication 4, ladite étape de détermination pour savoir si le module de capteur (110) est valide pour une utilisation comprenant en outre :
la détermination d'une période d'expiration du module de capteur (110) à partir de l'identifiant ou de la date de fabrication du module de capteur et la détermination pour savoir si la période d'expiration du module de capteur a expiré en comparant la période d'expiration du module de capteur (110) à la date courante, ou
la détermination pour savoir si le module de capteur (110) est défectueux en comparant l'identifiant ou le lot de production du module de capteur (110) avec une liste de défauts reçue du serveur (50).

6. Procédé selon la revendication 5, ladite étape d'initialisation et d'enregistrement du module de capteur (110) comprenant en outre :
en cas de détermination que la période d'expiration du module de capteur (110) a expiré ou que le module de capteur (110) est défectueux, la génération d'un message d'avertissement et l'activation et l'émission du message d'avertissement à destination du terminal utilisateur (200).

7. Procédé selon la revendication 6, ladite étape d'initialisation et d'enregistrement du module de capteur (110) comprenant en outre :
en cas de détermination qu'une commande de confirmation d'utilisateur pour le message d'avertissement activé est entrée, la transmission d'informations sur un temps d'activation du message d'avertissement, un temps d'entrée de la commande de confirmation d'utilisateur et l'identifiant du module de capteur correspondant (110) au serveur de gestion (50).

8. Procédé selon la revendication 3, ledit stockage des informations biométriques comprenant :
la comparaison des premières informations d'utilisateur fournies dans le message de mesure aux informations d'utilisateur enregistrées ;
la détermination pour savoir si les secondes informations d'utilisateur identiques aux premières informations d'utilisateur sont présentes dans les informations d'utilisateur enregistrées ; et
l'enregistrement et le stockage des informations biométriques fournies dans le message de mesure en mettant en correspondance les informations biométriques avec les secondes informations d'utilisateur identiques aux premières informations d'utilisateur.

9. Procédé selon la revendication 8, ladite étape d'enregistrement et de stockage des informations biométriques comprenant en outre :
en cas de détermination que les secondes informations d'utilisateur identiques aux premières informations d'utilisateur ne sont pas présentes dans les informations d'utilisateur enregistrées, la génération d'un message de confirmation d'utilisateur et l'activation et l'émission du message de confirmation d'utilisateur à destination du terminal utilisateur (200) .

10. Procédé selon la revendication 3, comprenant en outre le calcul d'une période d'utilisation restante en fonction d'informations d'heure de début d'utilisation et d'une période d'utilisation du module de capteur (110).

11. Procédé selon la revendication 10, comprenant en outre :
en cas d'entrée d'une commande de confirmation de période d'utilisation restante, le calcul d'une période d'utilisation restante conformément à une période d'utilisation réelle et à la période d'utilisation du module de capteur (110), la période d'utilisation réelle s'étendant de l'heure de début d'utilisation à une heure à laquelle la commande de confirmation de période d'utilisation restante est entrée, et l'émission d'informations sur la période d'utilisation restante à destination de l'utilisateur.

12. Procédé selon la revendication 10, comprenant en outre :
si la période d'utilisation restante est inférieure à un seuil défini, la génération d'un message de notification de remplacement et l'activation et l'émission du message de notification de remplacement généré à destination de l'utilisateur.
